(19) Europäisches Patentamt / European Patent Office / Office européen des brevets

(11) **EP 4 201 216 A1**

(12) **EUROPEAN PATENT APPLICATION**
published in accordance with Art. 153(4) EPC

(43) Date of publication:
**28.06.2023 Bulletin 2023/26**

(21) Application number: **21861576.3**

(22) Date of filing: **24.08.2021**

(51) International Patent Classification (IPC):
**A23C 11/10** $^{(2021.01)}$    **A23L 2/38** $^{(2021.01)}$
**A23L 7/10** $^{(2016.01)}$    **A23L 11/60** $^{(2021.01)}$
**A23L 25/00** $^{(2016.01)}$    **C12N 9/10** $^{(2006.01)}$

(52) Cooperative Patent Classification (CPC):
**A23C 11/10; A23L 2/38; A23L 7/10; A23L 11/60;**
**A23L 25/00; C12N 9/10**

(86) International application number:
**PCT/JP2021/031040**

(87) International publication number:
**WO 2022/045152 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.08.2020 JP 2020141158**

(71) Applicants:
• **Amano Enzyme Inc.**
**Nagoya-shi**
**Aichi 460-8630 (JP)**

• **Amano Enzyme Europe Ltd.**
**Oxfordshire, OX75SR (GB)**

(72) Inventor: **FUJIOKA, Hiroki**
**Chipping Norton, Oxfordshire, OX75SR (GB)**

(74) Representative: **Müller-Boré & Partner**
**Patentanwälte PartG mbB**
**Friedenheimer Brücke 21**
**80639 München (DE)**

(54) **VEGETABLE MILK TREATED WITH PROTEIN DEAMIDASE**

(57) The present invention addresses the problem of creating an effective means for preventing the coagulation of a plant milk under high temperature conditions. To solve this problem, a plant milk is treated with a protein deamidase to thereby prevent the coagulation of the plant milk in the case of adding to a hot liquid beverage, a hot liquid food, etc.

EP 4 201 216 A1

**Description**

TECHNICAL FIELD

The present invention relates to a plant milk treated with a protein deamidase. Specifically, the present invention relates to a plant milk that has been subjected to a treatment for improving dispersibility (that is, it is difficult to coagulate) when mixed with a heated liquid food or beverage, uses thereof, and the like.

BACKGROUND ART

On the background of allergy problems, an increase in the number of vegetarian people, religious reasons, and the like, plant-derived soybean proteins have become widespread as an alternative raw material for food beverages using an animal-derived milk protein source typified by cow's milk. Furthermore, in recent years, alternative raw materials have been diversified, and protein raw materials derived from plants other than soybean have been actively developed. In fact, proteins derived from cereals such as peas, rice, and oats, and nut proteins such as almonds, cashew nuts, and peanuts have been commercialized one after another as foods and beverages, similarly to soybean.

On the other hand, a plant milk such as almond milk has a property of coagulating in a heat and/or low-pH environment (Non-Patent Document 1). For this reason, for example, the problem of coagulation in actual use conditions of adding to high temperature acidic beverages such as coffee and black tea as a substitute for milk cannot be avoided.

PRIOR ART DOCUMENTS

NON-PATENT DOCUMENT

Non-Patent Document 1: "Rich almond milk (for commercial use)", [online], 2015, Tsukuba Dairy Products Co., Ltd., [searched on August 17, 2021], Internet <URL:https://www.tsukuba-milk.co.jp/almondmilkpage22>

SUMMARY OF THE INVENTION

PROBLEMS TO BE SOLVED BY THE INVENTION

To the best of the inventors' knowledge, there is no report clearly showing a measure against coagulation that occurs when a plant milk is added to a hot liquid beverage. Such coagulation is only dealt with among consumers to moderate the temperature change, for example, to reduce the temperature difference between the plant milk and the liquid beverage, or to slowly pour the liquid beverage into the plant milk, and has not reached an essential solution.

Therefore, an object of the present invention is to create an effective means for preventing coagulation of a plant milk used for producing a liquid food or beverage such as a beverage containing a plant milk in a heated state in order to increase the value of a plant milk and promote its use or application.

MEANS FOR SOLVING THE PROBLEM

In the course of repeated studies in view of the above problems, the present inventors have found that when a plant milk used for production of a liquid food or beverage such as a heated beverage is treated with a protein deamidase, dispersibility when the plant milk is added to a liquid food such as a heated beverage is improved (that is, coagulation is suppressed or prevented). The present invention has been completed by further conducting studies based on this finding. That is, the present invention provides inventions of the following aspects.

[1] A plant milk treated with a protein deamidase for mixing with a heated liquid food or beverage to prepare a plant milk-containing liquid food or beverage. (A plant milk treated with a protein deamidase for preparing a high temperature plant milk-containing liquid food or beverage.)

[2] The plant milk according to [1], wherein the plant milk is selected from the group consisting of nut milk, soy milk, pea milk, oat milk, hemp milk, and buckwheat milk.

[3] The plant milk according to [2], wherein nuts as a raw material of the nut milk are selected from the group consisting of almond, cashew nut, hazelnut, pecan nut, macadamia nut, pistachio, walnut, brazil nut, peanut, coconut, chestnut, sesame, and pine nut.

[4] The plant milk according to any one of [1] to [3], having a raw material plant protein concentration of 0.2% (w/v) to 10.0% (w/v).

[5] The plant milk according to any one of [1] to [4], wherein dispersibility when mixed with a heated liquid food or beverage is improved by the treatment.

[6] The plant milk according to [5], wherein pH of the liquid food or beverage is 3 or more and less than 11. (The plant milk according to [5], which does not cause protein coagulation when mixed with a weakly acidic to weakly alkaline liquid food or beverage (however, the pH of the mixed solution is 5 or more).)

[7] The plant milk according to [6], wherein the pH of the liquid food or beverage is 5 to 7.

[8] The plant milk according to [6], wherein the liquid food or beverage is selected from the group consisting of coffee, coffee beverages, tea, tea beverages, fruit juice, fruit juice beverages, sports beverages, nutritional beverages, soup, curry, cocoa, and chocolate beverages.

[9] The plant milk according to any one of [1] to [8], which does not contain an emulsifier and a thickening polysaccharide for preventing coagulation.

[10] The plant milk according to any one of [1] to [9], wherein the protein deamidase is an enzyme derived from a Chryseobacterium microorganism.

[11] The plant milk according to [10], wherein the Chryseobacterium microorganism is Chryseobacterium proteolyticum.

[12] A method for producing a plant milk having improved dispersibility when mixed with a heated liquid food or beverage, the method comprising a step of treating a plant milk with a protein deamidase.

[13] The production method according to [12], comprising the following steps (1) and (2):

(1) a step of preparing a plant milk, and
(2) a step of treating the plant milk prepared in (1) with a protein deamidase.

[14] The production method according to [13], comprising step (3) of performing a heating treatment after step (2) (The production method according to [13], further comprising the following step (3): (3) step of performing a heating treatment).

[15] A liquid food or beverage containing the plant milk according to any one of [1] to [11].

[16] The liquid food or beverage according to [15], wherein the pH is 5 or more.

[17] The liquid food or beverage according to [15] or [16], which is selected from the group consisting of coffee beverages, coffee whiteners, tea beverages, fruit juice beverages, sports beverages, nutritional beverages, soup, curry, cocoa beverages, and chocolate beverages.

[18] A method for producing a plant milk-containing liquid food or beverage, comprising a step of mixing a plant milk treated with a protein deamidase with a heated liquid food or beverage. (A method for producing a liquid food or beverage, comprising mixing a plant milk treated with a protein deamidase with a raw material, an intermediate product, or a final product of the liquid food or beverage under a high temperature condition.)

[19] The production method according to [18], comprising the following steps (1) and (2):

(1) a step of preparing a plant milk treated with a protein deamidase, and
(2) a step of mixing the plant milk prepared in (1) with the heated liquid food or beverage.

EMBODIMENTS OF THE INVENTION

1. Plant milk treated with protein deamidase

A first aspect of the present invention relates to a plant milk treated with a protein deamidase for mixing with a heated liquid food or beverage to prepare a plant milk-containing liquid food or beverage. That is, the plant milk of the present invention is a plant milk having improved dispersibility when added to a high temperature liquid food or beverage.

The plant milk of the present invention is improved in dispersibility when added to a high temperature liquid food or beverage by treating the plant milk with a protein deamidase. Hereinafter, for convenience of description, the plant milk in a state before being subjected to the treatment with the protein deamidase is also referred to as "untreated plant milk", the plant milk after being treated with the protein deamidase is also referred to as "plant milk of the present invention", and the characteristic of the plant milk of the present invention that improves dispersibility when mixed with a high temperature liquid food or beverage is also referred to as "predetermined improved dispersibility".

1-1. Untreated plant milk

The plant milk (That is, untreated plant milk) is a liquid in which a pulverized (is subdivided by a pulverizing means such as compression pulverization, impact pulverization, shear pulverization, or grinding) product of a raw material plant (Specifically, the plant edible part) containing a plant protein is dispersed in water, and the plant edible part is not particularly limited, and examples thereof include various nuts, soybean, oats, peas, hemp, lupin bean, broad bean, chick bean, barley, wheat, rice, buckwheat, barnyard millet, awa millet, canary seed, teff, quinoa, linseed, and the like. Specific examples of the various nuts are not particularly limited, but examples thereof include almond, cashew nut, hazelnut, pecan nut, macadamia nut, pistachio, walnut, brazil nut, peanut, coconut, chestnut, sesame, and pine nut.

In the untreated plant milk, the raw material plants may be used alone, or two or more kinds thereof may be used in combination (examples thereof include combined use of almond and cashew nut, combined use of almond and peanut, and the like).

The method for preparing an untreated plant milk is not particularly limited, and generally, a person skilled in the art can appropriately combine steps such as grinding, immersion, dissolution, mixing, stirring, filtration, homogenization, and sterilization of the plant edible part. The method for preparing nut milk is also not particularly limited, and generally, a person skilled in the art can appropriately combine steps such as water immersion, dissolution, mixing, stirring, filtration, and homogenization, sterilization using the de-nucleated nuts. As the untreated plant milk, a plant milk provided by a raw material manufacturer, for example, a commercially available plant milk may be purchased and used in the present invention.

The protein concentration (raw material plant protein concentration) in the untreated plant milk is not particularly limited, but an untreated plant milk having a protein concentration of, for example, 0.2% (w/v) to 10.0% (w/v), preferably 0.2% (w/v) to 8.0% (w/v), more preferably 0.2% (w/v) to 5.0% (w/v) is used. The protein concentration of the plant milk of the present invention after the protein deamidase treatment is also, for example, 0.2% (w/v) to 10.0% (w/v), preferably 0.2% (w/v) to 8.0% (w/v), more preferably 0.2% (w/v) to 5.0% (w/v).

1-2. Protein deamidase

The protein deamidase used for the treatment of an untreated plant milk has an action of directly acting on the amide group of the protein to perform deamidation without cleavage of peptide bonds and crosslinking of the protein. The type, origin, and the like of the enzyme are not particularly limited as long as the enzyme exhibits the above action. Examples of the protein deamidase include protein deamidases derived from the genus Chryseobacterium, Flavobacterium, Empedobacter, Sphingobacterium, Aureobacterium, or Myroides, which are disclosed in JP 2000-50887 A, JP 2001-218590 A, WO 2006/075772, and the like, and commercially available protein glutaminase derived from the genus Chryseobacterium, and the like. Preferably, an enzyme derived from the genus Chryseobacterium (specific examples thereof include an enzyme derived from Chryseobacterium proteolyticum (for example, protein glutaminase "Amano " 500 manufactured by Amano Enzyme Inc.)) is used.

As the protein deamidase, one prepared from a culture solution of a microorganism producing the protein deamidase can be used. The microorganism to be used for the preparation of the protein deamidase is not particularly limited, but a microorganism that produces the enzyme, for example, a microorganism belonging to the genus Chryseobacterium, Flavobacterium, Empedobacter, Sphingobacterium, Aureobacterium, or Myroides can be used. Specific examples of microorganisms suitable for preparation of the protein deamidase include Chryseobacterium sp. No. 9670 belonging to the genus Chryseobacterium.

For example, a protein deamidase can be obtained from a culture solution or a bacterial cell of the above-mentioned microorganism. That is, the secreted protein can be recovered from the culture solution, and the other proteins can be recovered from the bacterial cells. As a method for preparing the protein deamidase from the culture solution, a known protein separation and purification method (Centrifugation, UF concentration, salting-out, various types of chromatography using an ion exchange resin or the like, etc.) can be used. For example, the culture solution is centrifuged to remove bacterial cells, and then salting-out, chromatography, and the like can be combined to obtain a target enzyme. In the case of recovering the enzyme from the bacterial cells, for example, the bacterial cells are crushed by pressurization treatment, ultrasonic treatment, or the like, and then separated and purified in the same manner as described above, whereby the target enzyme can be obtained. The above-mentioned series of steps (Disruption, separation, and purification of bacterial cells) may be carried out after the bacterial cells are previously recovered from the culture solution by filtration, centrifugation or the like. The enzyme may be pulverized by a drying method such as freeze drying and reduced pressure drying, or an appropriate excipient or drying aid may be used at that time.

In the present application, the activity of the protein deamidase is measured by the following method.

(1) Here, 0.1 ml of an aqueous solution containing a protein deamidase is added to 1 ml of a 0.2 M phosphate buffer (pH 6.5) containing 30 mM Z-Gln-Gly, and the mixture is incubated at 37°C for 10 minutes, then 1 ml of a 0.4 M TCA solution is added to stop the reaction. As a blank, 0.1 ml of an aqueous solution containing a protein deamidase is added to a mixture obtained by adding 1 ml of a 0.2 M phosphate buffer (pH 6.5) containing 30 mM Z-Gln-Gly and 1 ml of a 0.4 M TCA solution, and the mixture is incubated at 37°C for 10 minutes.

(2) The amount of ammonia generated by the reaction is measured using Ammonia Test Wako (FUJIFILM Wako Pure Chemical Corporation) for the solution obtained in (1). The ammonia concentration in the reaction liquid is determined from a calibration curve representing the relationship between the ammonia concentration and the absorbance (630 nm) prepared using an ammonia standard solution (ammonium chloride).

(3) The activity of the protein deamidase is calculated from the following formula, with the amount of enzyme producing 1 μmol ammonia per minute as one unit.

$$\text{Enzyme activity (U/mL)} = \text{ammonia concentration in reaction liquid (mg/L)} \times$$

$$(1/17. \quad 03) \times (\text{amount of reaction liquid/amount of enzyme solution}) \times (1/10) \times Df$$

(In the formula, the reaction liquid amount is 2.1, the enzyme solution amount is 0.1, and Df is a dilution rate of the enzyme solution. Further, 17.03 is a molecular weight of ammonia.)

1-3. Treatment conditions with protein deamidase

As long as the predetermined improved dispersibility can be imparted to the plant milk, the conditions of the treatment with the protein deamidase are not particularly limited, and it is only required that those skilled in the art appropriately adjust the reaction temperature, the reaction time, and the amount of the enzyme to be added (enzyme concentration), and set the optimum reaction conditions.

Although not limited, the reaction temperature may be set, for example, within a range of 2°C to 70°C, preferably within a range of 5°C to 60°C, and more preferably within a range of 15°C to 50°C. Similarly, the reaction time may be set, for example, within a range of 10 minutes to 7 days, preferably within a range of 30 minutes to 3 days, and more preferably within a range of 1 hour to 1 day. The amount of the enzyme to be added may be set, for example, within a range of 0.01 (U/g protein) to 500 (U/g protein), preferably within a range of 0.02 (U/g protein) to 50 (U/g protein), and more preferably within a range of 0.2 (U/g protein) to 5 (U/g protein). Here, the "U/g protein" is the number of units per 1 g of the raw material plant protein serving as a substrate.

Here, when the treatment conditions with the protein deamidase are set, in a case where the reaction for improving dispersibility is further promoted, the reaction temperature can be increased, the reaction time can be lengthened, and/or the amount of the enzyme to be added can be increased, and in a case where the reaction for improving dispersibility is more slowed, the reaction temperature can be lowered, the reaction time can be shortened, and/or the amount of the enzyme to be added can be reduced. When the degree of acceleration or slowness of the reaction for improving dispersibility is more finely controlled, the following indexes (a) to (c) may be followed.

(a) When the reaction temperature is lowered, the reaction time is lengthened, the amount of the enzyme to be added is increased, or both of them are combined.

(b) In the case of shortening the reaction time, the reaction temperature is increased (however, the temperature is not higher than 70°C, preferably 60°C or lower), the amount of the enzyme to be added is increased, or both of them are combined.

(c) When the amount of the enzyme to be added is reduced, the reaction temperature is increased (however, the temperature is not higher than 70°C, preferably 60°C or lower) or the reaction time is lengthened (or both of them are combined).

The indexes (a) to (c) can also be used when various conditions are set according to the specifications of the reaction facility or the like while the degree of acceleration or slowness of the reaction for improving dispersibility is maintained.

A more specific index for setting the processing condition is exemplified below.

In the case of 5 °C ≤ reaction temperature < 15°C, the reaction time is set to more than 8 hours (preferably 24 hours or more), or the amount of the enzyme to be added is set to 0.2 (U/g protein) or more (preferably 1 (U/g protein) or more).

In the case of 15°C ≤ reaction temperature < 25 °C, the reaction time is set to more than 7 hours, or the amount of the enzyme to be added is to more than 0.2 (U/g protein) (preferably 1 (U/g protein) or more).

In the case of 25°C ≤ reaction temperature < 40°C, the reaction time is set to more than 5 hours (preferably 7 hours or more), or the amount of the enzyme to be added is set to 0.2 (U/g protein) or more (preferably 1 (U/g protein) or more).

In the case of 40°C ≤ reaction temperature < 50°C, the reaction time is set to preferably 3 hours or more, or the amount of the enzyme to be added is set to preferably 0.2 (U/g protein) or more.

In the case of 50 ≤ reaction temperature (however, the temperature is not higher than 70°C, preferably 60°C or lower), the reaction time is set to preferably 3 hours or more, or the amount of the enzyme to be added is set to preferably 0.2 (U/g protein) or more.

1-4. Predetermined improved dispersibility and applications

As described above, the plant milk of the present invention has predetermined improved dispersibility. That is, the plant milk of the present invention is excellent in dispersibility when added to a high temperature liquid food or beverage.

Having predetermined improved dispersibility means exhibiting improved dispersibility when mixed with a high temperature liquid food or beverage. The high temperature exhibited by the liquid food or beverage means that the temperature is high enough to cause protein coagulation of the untreated plant milk by heat when the liquid food or beverage is mixed with an untreated plant milk. The improved dispersibility refers to dispersibility improved as compared with dispersibility when the liquid food or beverage is mixed with an untreated plant milk (that is, dispersibility poor enough to cause coagulation). In the present invention, the dispersibility and the coagulation suppressing property are used synonymously.

The specific temperature of the high temperature is not particularly limited as long as it is a temperature at which protein coagulation of the untreated plant milk occurs due to heat when the liquid food or beverage is mixed with the untreated plant milk, and it may be at least a temperature exhibited by heating of the liquid food or beverage. Specific examples of the high temperature are, for example, 50°C or higher, preferably 60°C or higher, further preferably 70°C or higher, more preferably 80°C or higher, and most preferably 90°C or higher. The upper limit of the high temperature is, for example, 100°C.

As described above, since the plant milk of the present invention is excellent in dispersibility when added to a high temperature liquid food or beverage, it is used for the purpose of preparing a high temperature plant milk-containing liquid food or beverage, that is, for the purpose of preparing a plant milk-containing liquid food or beverage by mixing with a heated liquid food or beverage.

The temperature of the plant milk of the present invention when used for mixing with the liquid food or beverage is not particularly limited as long as the temperature of the liquid food or beverage (That is, the plant milk-containing liquid food or beverage) when the plant milk of the present invention is mixed is a temperature that satisfies the above-described high temperature condition (that is, the heating condition is, for example, 50°C or higher).

The liquid property of the liquid food or beverage with which the plant milk of the present invention is mixed at the time of use is not particularly limited, and may be not only neutral but also weakly acidic. That is, the pH of the liquid food or beverage is typically 3 or more and less than 11, that is, weakly acidic ($3 \leq pH < 6$) to weakly alkaline ($8 \leq pH < 11$), and preferably weakly acidic $3 \leq pH < 6$. The pH is a pH at a temperature when the liquid food or beverage is mixed with the plant milk treated with a protein deamidase.

The pH of the mixture of the plant milk of the present invention and a liquid food or beverage (That is, the plant milk-containing liquid food or beverage) is, for example, 5 or more, preferably 5 to 10, more preferably 5 to 9, still more preferably 5 to 8, even more preferably 5 to 7.5, and particularly preferably 5 to 7. The pH is a pH at a temperature when the liquid food or beverage and the plant milk treated with a protein deamidase are mixed (that is, when a plant milk-containing liquid food or beverage is obtained).

Examples of the liquid food or beverage with which the plant milk of the present invention is mixed at the time of use include beverages and liquid foods. The beverage refers to a liquid (What can itself be a final product form) ingested by the act of directly drinking itself, and typically includes recreational beverages. The liquid food refers to a fluid dish (what can itself be a final product form), or a liquid intermediate product or raw material (raw materials also include additives) used for producing the beverage or the dish.

Specific examples of the beverage include coffee, coffee beverages, tea (tea extract such as black tea, green tea, or oolong tea, reduction of the extract (that is, dilution with water), and reduction of the extract after processing (e.g., concentrated, lyophilized) (that is, dilution by dissolution in water)), tea beverages (Flavored tea, milk tea, tea beverage containing fruit juice, and the like), fruit juice, fruit juice beverages, cocoa, chocolate beverages, sports beverages, and the like. Specific examples of the liquid food include nutritional beverages (protein beverages, nursing care nutritional beverages, etc.), soup (bouillon-based soup, stew, chowder, borscht, vegetable soup (for example, tomato soup, corn soup, potage, or pumpkin soup), and miso-soup), curry, and the like as final products, and include the same as the above-described beverages and coffee whiteners (added to coffee and citations other than coffee (for example, black tea, etc.)) as intermediate products or raw materials, and the like.

In a preferred aspect of the liquid food or beverage to be mixed at the time of use of the plant milk of the present invention, the plant milk of the present invention does not contain an additive for preventing coagulation (also described as a coagulation inhibitor), specifically, an emulsifier (glycerin fatty acid ester, sucrose fatty acid ester, lecithin, saponin, and the like), a thickening polysaccharide (pectin, carboxymethyl cellulose, etc.), salts (common salts (seasalt), calcium salts, phosphate salts, and the like), and the like, taking advantage of the characteristics that the plant milk of the present invention exhibits excellent dispersibility and is less likely to cause protein coagulation when mixed with a high temperature liquid food or beverage. In particular, the liquid food or beverage preferably does not contain an emulsifier and a thickening polysaccharide. In this preferred embodiment, use of an additive added for a purpose other than prevention of coagulation is allowed. Examples of the additive added for the purpose other than the prevention of coagulation include a seasoning, a preservative, a flavor, an antioxidant, and the like for the purpose of adjusting the taste and/or flavor. As described above, according to the plant milk of the present invention, it is possible to prepare a plant milk-containing liquid food or beverage that does not use a coagulation inhibitor in a state in which coagulation is suppressed, and thus it is possible to meet the needs of consumers for products with less additives or without additives.

2. Method for producing plant milk with improved dispersibility when mixed with heated liquid food or beverage

A second aspect of the present invention relates to a method for producing a plant milk having improved dispersibility when mixed with a heated liquid food or beverage. As described above, a plant milk having predetermined improved dispersibility can be produced by treating an untreated plant milk with a protein deamidase. Therefore, the present invention also provides a method for producing a plant milk having improved dispersibility when mixed with a heated liquid food or beverage, the method including a step of treating a plant milk (untreated plant milk) with a protein deamidase.

Typically, the method for producing a plant milk with improved dispersibility of the present invention includes the following steps (1) and (2).

(1) a step of preparing a plant milk (That is, untreated plant milk); and
(2) a step of treating the plant milk prepared in (1) with a protein deamidase.

Specific embodiments in step (1) include the case of preparing a plant milk and the case of preparing a commercially available plant milk. The method for preparing a plant milk is as described above in "1-1. Untreated plant milk". The plant milk prepared in step (1) may be heat-sterilized or may not be heat-treated.

The protein deamidase used in step (2) is as described above in "1-2. Protein deamidase", and the treatment conditions are as described above in "1-3. Treatment conditions with protein deamidase".

The method for producing a plant milk with improved dispersibility of the present invention may further include heating treatment step (3) before or after step (2).

Specifically, when step (3) is included before step (2), step (2) can be performed after the untreated plant milk prepared in step (1) is heat-sterilized by heating treatment. When step (3) is included after step (2), the protein deamidase-treated plant milk obtained in step (2) can be heat-treated. When step (3) is included after step (2), step (3) can also serve as heat deactivation of the protein deamidase used in step (2), in addition to heat sterilization of the protein deamidase-treated plant milk obtained in step (2).

In other words, step (2) may be performed either before or after heat sterilization of the plant milk. However, in order to simplify the production process, it is preferable to perform the step before heat sterilization of the plant milk, and then to perform a heat sterilization step that also serves as deactivation of the protein deamidase. Therefore, in a preferred aspect, step (3) is performed after step (2).

When step (3) is performed before step (2), the condition of the heating treatment in step (3) is not particularly limited as long as the sterilization of the untreated plant milk is possible, and when step (3) is performed after step (2), the condition of the heating treatment is not particularly limited as long as at least one of the deactivation of the protein deamidase and the sterilization of the plant milk subjected to the protein deamidase treatment is possible. In any case, the specific condition of step (3) is, for example, 1 second to 5 hours at a temperature of 70°C to 150°C.

By the above method, the plant milk having the predetermined improved dispersibility described in the above "1. Plant milk treated with protein deamidase" is obtained.

3. Plant milk-containing liquid food or beverage

A third aspect of the present invention relates to a plant milk-containing liquid food or beverage. The plant milk having the predetermined improved dispersibility described in the above "1. Plant milk treated with protein deamidase" is excellent in dispersibility when mixed with a liquid food or beverage under high temperature conditions, and is less likely to cause protein coagulation. Therefore, the plant milk having the predetermined improved dispersibility is used for preparing a plant milk-containing liquid food or beverage at a high temperature. That is, the plant milk having the predetermined improved dispersibility can be applied to a plant milk-containing liquid food or beverage having excellent dispersibility. Therefore, the present invention also provides a liquid food or beverage containing the plant milk having the predetermined improved dispersibility described above in "1. Plant milk treated with protein deamidase".

The plant milk-containing liquid food or beverage of the present invention may contain a liquid food or beverage and a plant milk having a predetermined improved dispersibility. Details of the pH, form, specific example, acceptable additives, and the like of the liquid food or beverage, details of the pH, and the like of the plant milk-containing liquid food or beverage, and dispersion characteristics (predetermined improved dispersibility) of the plant milk in the plant milk-containing liquid food or beverage are as described in "1-4. Predetermined improved dispersibility and applications".

## 4. Method for producing plant milk-containing liquid food or beverage

A fourth aspect of the present invention relates to a method for producing a plant milk-containing liquid food or beverage. The plant milk treated with a protein deamidase is mixed with other raw materials (liquid food or beverage) in the middle of the production process of the plant milk-containing liquid food or beverage, for example. Therefore, the present invention also provides a method for producing a plant milk-containing liquid food or beverage, including a step of mixing a plant milk treated with a protein deamidase with a heated liquid food or beverage.

Preferably, the protein deamidase plant milk can be finally mixed at the final stage of the production process of the plant milk-containing liquid food or beverage, that is, after all of mixing and processing of other raw materials other than the plant milk have been performed (stage of being in the final product form). Thereafter, sterilization treatment and/or addition of an additive (preferably, additives other than the coagulation inhibitor, such as seasoning, preservatives, flavors, and antioxidants for the purpose of adjusting the taste, maintaining the quality, and the like) may be further performed. On the other hand, it is also a preferable aspect that the plant milk is mixed with a liquid food or beverage (that is, it is not in the form of a final product but in the form of an intermediate product or a raw material) in the middle of the production process in which not all of the mixing and processing of other raw materials other than the plant milk are performed.

The method for producing a plant milk-containing liquid food or beverage of the present invention typically includes the following steps (1) and (2).

(1) a step of preparing a protein deamidase-treated plant milk, and
(2) a step of mixing the plant milk prepared in (1) with a heated liquid food or beverage.

When the plant milk subjected to the protein deamidase treatment in step (1) is prepared, the protein deamidase to be used is as described above in "1-2. Protein deamidase", the plant milk is as described above in "1-1. Untreated plant milk", and the conditions of the enzyme treatment are as described above in "1-3. Treatment conditions with protein deamidase". The liquid food or beverage used in step (2) is as described in "1-4. Predetermined improved dispersibility and applications".

According to the above method, the plant milk-containing liquid food or beverage described in the above "3. Plant milk-containing liquid food or beverage" is obtained.

## EXAMPLES

Test Example 1. Prevention of coagulation in coffee

To 100 mL of commercially available almond milk (manufactured by Rude Health, protein content: 1.5 w/v%, raw material: almond, water, subjected to ultra-high temperature heating treatment), protein glutaminase "Amano " 500 (manufactured by Amano Enzyme Inc., 500 U/g) was added in an amount of 1 U per g of protein in almond milk, and a reaction was caused at 50°C for 5 hours (deamidation reaction). The enzyme was heat-inactivated by treatment at 95°C for 20 minutes, and then cooled to 5 °C to give enzyme-treated almond milk.

Commercial instant coffee was dissolved in hot water to prepare a coffee liquid (2 w/v%, pH 5.2, 90°C). When 20 to 30 mL (5°C) of the enzyme-treated almond milk was added to 150 mL (90°C) of the coffee liquid (pH of the coffee liquid after addition of the enzyme-treated almond milk was 5.7), coagulation was not observed. In contrast, clear coagulation was observed when non-enzyme-treated almond milk was used. An experiment was conducted under the same conditions except that peanut milk was used instead of almond milk, and the results were similar (no coagulation occurred in the coffee liquid to which the enzyme-treated peanut milk was added, and obvious coagulation was observed in the coffee liquid to which the non-enzyme-treated peanut milk was added).

Test Example 2. Relationship between protein concentration of nut milk and coagulation/coagulation preventing effect

(1) Methods

<No enzyme treatment>

Commercially available almond milk (manufactured by Rude Health, protein content: 1.5 w/v%, raw material: almond, water, subjected to ultra-high temperature heating treatment) was diluted with tap water as necessary so as to have protein concentrations of 0.1, 0.5, and 1.5% (w/v), then cooled to 5°C, and 5 mL of each was added to 50 mL of a coffee solution (pH: 5.2) warmed to 90°C to confirm whether coagulation occurred.

<Enzyme treatment present>

To commercially available almond milk (manufactured by Rude Health, protein content: 1.5 w/v%, raw material: almond, water, subjected to ultra-high temperature heating treatment), protein glutaminase "Amano" 500 (manufactured by Amano Enzyme Inc., 500 U/g) was added in an amount of 1 U per g of protein in almond milk, and a reaction was caused at 50°C for 5 hours (deamidation reaction). The enzyme was heat-inactivated by treatment at 90°C for 15 minutes to give enzyme-treated almond milk. The enzyme-treated almond milk was diluted with tap water to a protein concentration of 0.1, 0.5, 0.75, 1.0, 1.5% (w/v) and then cooled to 5°C, and to 50 mL of a coffee solution (pH: 5.2) warmed to 90°C, 5 mL of each was added to confirm whether coagulation occurred.

(2) Results

The results are shown in Table 1. As shown in Table 1, apparent coagulation was observed when the almond milk without enzyme treatment was added to the coffee liquid, whereas coagulation was not observed when the almond milk with enzyme treatment was added to the coffee liquid. That is, the almond milk having been subjected to the enzyme treatment could acquire the property of improving the dispersibility when mixed with a high temperature liquid food or beverage.

[Table 1]

| Protein concentration | Without enzyme treatment | With enzyme treatment |
|---|---|---|
| 0.1% | Coagulated | No coagulation |
| 0.5% | Coagulated | No coagulation |
| 0.75% | | No coagulation |
| 1.0% | | No coagulation |
| 1.5% | Coagulated | No coagulation |

Test Example 3. Relationship between pH of liquid and coagulation/coagulation preventing effect

(1) Methods

Here, 15 to 20 mL of non-enzyme-treated almond milk or enzyme-treated almond milk (protein concentration: 1.5% (w/v)) was added to hot water that had been heated to 90°C and whose pH had been adjusted with hydrochloric acid or sodium hydroxide, and coagulation was confirmed. The enzyme-treated almond milk was prepared by the method described in the experiment of Test Example 2 above. The pH of the hot water containing the non-enzyme-treated almond milk or the enzyme-treated almond milk is as shown in Table 2.

(2) Results

The results are shown in Table 2. As shown in Table 2, apparent coagulation was observed when the almond milk without enzyme treatment was added to the hot water, whereas coagulation was not observed when the almond milk with enzyme treatment was added to the hot water. That is, the almond milk having been subjected to the enzyme treatment could acquire the property of improving the dispersibility when mixed with a high temperature liquid food or beverage.

[Table 2]

| pH after addition | Without enzyme treatment |
|---|---|
| 5.4 | Coagulated |
| 5.9 | Coagulated |
| 6.6 | Coagulated |
| 7 | Coagulated |

(continued)

| pH after addition | With enzyme treatment |
|---|---|
| 5 | No coagulation |
| 5.4 | No coagulation |
| 6.3 | No coagulation |
| 6.7 | No coagulation |
| 7.2 | No coagulation |

Test Example 4. Effect of preventing coagulation in various liquid foods and beverages

(1) Methods

<Black tea (straight tea)>

Boiling water was poured into a commercially available black tea pack (manufactured by Twining Inc., English Breakfast), and the mixture was extracted for 2 to 3 minutes, and then the tea pack was taken out to prepare black tea. Non-enzyme-treated almond milk or enzyme-treated almond milk (protein concentration 1.5% (w/v)) was added to this black tea, and the presence or absence of coagulation was confirmed. The black tea immediately before addition of the almond milk had a temperature of 80°C and a pH of 5.2. The pH of the black tea after addition of the almond milk was 5.9. The enzyme-treated almond milk was prepared by the method described in the experiment of Test Example 2 above.

<Lemon tea>

Boiling water was poured into a commercially available black tea pack (manufactured by Twining Inc., English Breakfast), and the mixture was extracted for 2 to 3 minutes, and then the tea pack was taken out to prepare black tea. Lemon juice was added to the black tea to adjust the pH, and then non-enzyme-treated almond milk or enzyme-treated almond milk (protein concentration: 1.5% (w/v)) was added to confirm whether coagulation occurred. The black tea immediately before addition of the almond milk had a temperature of 70°C and a pH of 4.0. The enzyme-treated almond milk was prepared by the method described in the experiment of Test Example 2 above.

<Decaf>

Boiling water was poured into a commercially available decaf coffee powder (manufactured by Nestlé Corporation, Nescafe Gold) to dissolve well, thereby preparing a decaf coffee solution. Non-enzyme-treated almond milk or enzyme-treated almond milk (protein concentration 1.5% (w/v)) was added thereto, and the presence or absence of coagulation was confirmed. The decaf coffee solution immediately before addition of the almond milk had a temperature of 80°C and a pH of 5.3. In addition, the pH of the decaf coffee solution after addition of the almond milk was 5.8. The enzyme-treated almond milk was prepared by the method described in the experiment of Test Example 2 above.

<Tomato soup>

A predetermined amount of boiling water was poured into a commercially available chicken soup stock (Knorr Chicken Broth Cube manufactured by Unilever) to completely dissolve the stock, thereby preparing a chicken soup, and then a commercially available tomato puree was added thereto. The pH of the tomato soup was adjusted by increasing or decreasing the amount of puree to be added, and then non-enzyme-treated almond milk or enzyme-treated almond milk (protein concentration: 1.5% (w/v)) was added to confirm the presence or absence of coagulation. The tomato soup immediately before addition of the almond milk had a temperature of 80°C and a pH of 5.0. The enzyme-treated almond milk was prepared by the method described in the experiment of Test Example 2 above.

(2) Results

The results are shown in Table 3. As shown in Table 3, for any liquid food or beverage, obvious coagulation was observed when the almond milk without enzyme treatment was added (the degree of coagulation in black tea was smaller than that in other liquid foods and beverages in the present Test Example), whereas coagulation was not observed when the almond milk with enzyme treatment was added. That is, the almond milk having been subjected to the enzyme treatment could acquire the property of improving the dispersibility when mixed with a high temperature liquid food or beverage.

[Table 3]

|  | pH after addition | Without enzyme treatment |
|---|---|---|
| Black tea | 5.9 | Coagulated |
| Decaf | 5.8 | Coagulated |
| Tomato soup | 5.4 | Coagulated |
| Lemon tea | 4.9 | Coagulated |

|  | pH after addition | With enzyme treatment |
|---|---|---|
| Black tea | 5.9 | No coagulation |
| Decaf | 5.8 | No coagulation |
| Tomato soup | 5.4 | No coagulation |
| Lemon tea | 5 | No coagulation |

Test Example 5. Effect of preventing coagulation of nut milk other than almond milk

(1) Methods

To commercially available peanut milk (manufactured by Rude Health, protein content: 2.0 w/v%, raw material: peanut, water, ultrahigh temperature heat-treated), and commercially available cashew nut milk (manufactured by PLENISH, protein content: 0.9 w/v%, raw materials: water, cashew nut, common salt, subjected to ultrahigh temperature heating treatment), pistachio milk (manufactured by Borna Food, protein content 1.0 w/v%, ultrahigh temperature heat-treated), and hazelnut milk (manufactured by Plenish, protein content: 0.6 w/v%, ultrahigh temperature heat-treated), was added protein glutaminase "Amano" 500 (manufactured by Amano Enzyme Inc., 500 U/g) in an amount of 1 U per g of the nut protein, and a reaction was caused at 50°C for 5 hours (deamidation reaction). After the enzyme reaction, the enzyme was rapidly deactivated by treatment at 90°C for 15 minutes, and cooled in flowing water, and then cooled in a refrigerator to 5°C, and thereafter, 5 mL of each was added to 50 mL of a coffee solution (pH: 5.2) warmed to 90°C, and the presence or absence of coagulation was confirmed.

(2) Results (Table 4)

In all of peanut milk, cashew nut milk, pistachio milk, and hazelnut milk, coagulation was observed when added to the coffee solution without enzyme treatment, but coagulation was not observed when added to the coffee solution in the case of enzyme treatment. This result indicates that the same effect by the enzyme treatment can be obtained also for nut milk other than almond milk, that is, all of peanut milk, cashew nut milk, pistachio milk, and hazelnut milk with the enzyme treatment have acquired the characteristic of improving dispersibility when mixed with a high temperature liquid food or beverage.

[Table 4]

|  | Without enzyme treatment | With enzyme treatment |
|---|---|---|
| Almond milk | Coagulated | No coagulation |
| Peanut milk | Coagulated | No coagulation |
| Cashew nut milk | Coagulated | No coagulation |
| Pistachio milk | Coagulated | No coagulation |
| Hazelnut milk | Coagulated | No coagulation |

Test Example 6. Relationship between temperature of liquid and coagulation/coagulation preventing effect

(1) Methods

<Changing coffee temperature (almond milk is constant at 5°C)>

To 50 mL of coffee (pH 5.0 to 5.3, 90°C) adjusted to the temperature shown in Table 5, 5 mL of non-enzyme-treated almond milk or enzyme-treated almond milk cooled to 5 °C was added, and the presence or absence of coagulation was confirmed. The enzyme-treated almond milk was prepared by the method described in the experiment of Test Example 2 above.

<Changing coffee temperature (almond milk is constant at 90°C)>

To 50 mL of coffee (pH 5.0 to 5.3, 90°C) adjusted to the temperature shown in Table 5, 5 mL of non-enzyme-treated almond milk or enzyme-treated almond milk warmed to 90°C was added, and the presence or absence of coagulation was confirmed. The enzyme-treated almond milk was prepared by the method described in the experiment of Test Example 2 above.

(2) Results

The results are shown in Table 5. As shown in Table 5, coagulation was observed when non-enzyme-treated almond milk was added to heated coffee. On the other hand, even in the heated coffee, coagulation was not observed when the enzyme-treated almond milk was added. That is, the enzyme-treated almond milk had acquired the property of improving the dispersibility when mixed with a high temperature (heated) liquid food or beverage.

[Table 5]

| Coffee temperature | Almond milk temperature | Without enzyme treatment | With enzyme treatment |
|---|---|---|---|
| (No heating) | 5°C | No coagulation | |
| 60°C | 5°C | Coagulated | No coagulation |
| 90°C | 5°C | Coagulated | No coagulation |
| (No heating) | 90°C | No coagulation | No coagulation |
| 90°C | 90°C | Coagulated | No coagulation |

Test Example 7 Study of enzyme treatment conditions (enzyme addition amount, reaction temperature, reaction time)

(1) Methods

To commercially available almond milk (manufactured by Rude Health, protein content: 1.5 w/v%, raw material: almond, water, subjected to ultra-high temperature heating treatment), protein glutaminase "Amano" was added in an amount shown in Table 6 per g of protein in the almond milk, and a reaction was caused at a temperature and for a time shown in Table 6 (deamidation reaction). After the enzyme reaction, the enzyme was rapidly deactivated by treatment at 90°C for 15 minutes, and cooled in flowing water, and then cooled in a refrigerator to 5°C, and thereafter, 5 mL of each was added to 50 mL of a coffee solution (pH 5.2) warmed to 90°C, and the presence or absence of coagulation was confirmed.

(2) Results

The results are shown in Table 6. Coagulation could be prevented regardless of the amount of the enzyme to be added, the reaction temperature, and the reaction time as conditions for the enzyme treatment of the almond milk. That is, it is found that the enzyme treatment conditions for obtaining the property of preventing coagulation when the almond milk is added to the coffee solution are allowed in a wide range by adjusting these conditions. Specifically, when the reaction temperature is low, a higher dispersibility improving effect can be obtained by increasing the amount of the enzyme to be added or increasing the reaction time (or both). For example, even when the reaction temperature was 5°C, a higher dispersibility improving effect was obtained when the amount of the enzyme added was 1 U or more or the reaction was performed for a long time. On the other hand, when the reaction time was short, by increasing the reaction temperature or increasing the amount of the enzyme added (or both), a higher dispersibility improving effect was obtained. For example, even when the reaction time was 3 hours, a higher dispersibility improving effect was obtained when the reaction temperature was set to 40°C or higher or when the amount of the enzyme added was 1 U or more. In addition, if the reaction temperature is increased or the reaction time is lengthened, or both, the amount of the enzyme to be added can be reduced. For example, when the reaction temperature is 25°C or higher or the reaction time is prolonged, the amount of the enzyme to be added can be 0.2 U or less.

[Table 6]

| Reaction temperature | Reaction time [hr] | Enzyme addition amount [U/g protein] | | |
|---|---|---|---|---|
| | | 0.2 U | 1U | 5 U |
| 5°C | 5 | | No coagulation | No coagulation |
| | 8 | | No coagulation | No coagulation |
| | 24 | No coagulation | No coagulation | No coagulation |
| 15°C | 3 | | No coagulation | No coagulation |
| | 5 | | No coagulation | No coagulation |
| | 7 | | No coagulation | No coagulation |
| 25°C | 3 | | No coagulation | No coagulation |
| | 5 | | No coagulation | No coagulation |
| | 7 | No coagulation | No coagulation | No coagulation |
| 40°C | 3 | No coagulation | No coagulation | No coagulation |
| | 5 | No coagulation | No coagulation | No coagulation |
| | 7 | No coagulation | No coagulation | No coagulation |
| 50°C | 3 | No coagulation | No coagulation | No coagulation |
| | 5 | No coagulation | No coagulation | No coagulation |
| | 7 | No coagulation | No coagulation | No coagulation |

<Summary>

- In particular, in the range of a nut protein concentration of 0.1 to 1.5% (w/v), a high dispersibility improving effect was observed when the nut protein was mixed with the heated liquid food or beverage regardless of the concentration. That is, it was shown that the enzyme treatment with the protein deamidase is effective for preventing coagulation of nut milk with various protein concentrations, and versatility is high.
- Although depending on the type of the liquid to be mixed with the nut milk, as a tendency, it has been found that the nut milk is strongly coagulated particularly when the pH is 7 or less after being mixed with the nut milk without the enzyme treatment by the protein deamidase, but coagulation can be suppressed even when the nut milk is subjected to the enzyme treatment at pH 5. Since the coagulation can be suppressed even at such a low pH, it was shown that the present invention can be applied to acidic liquid foods and beverages such as sour milk soup in addition to beverages such as coffee and black tea as liquid foods and beverages. In addition, since the milk lemon tea, which is difficult to prepare even when milk is used, could be easily prepared with high dispersibility according to the present invention, it was shown that the milk lemon tea can also be applied to a liquid food or beverage using a fruit having a sour taste.
- Enzyme treatment conditions (enzyme addition amount (enzyme concentration), reaction temperature, reaction time) of the plant milk for imparting predetermined improved dispersibility are allowed to be in a wide range if appropriately adjusted.
- Not only almond milk but also nut milk such as peanut milk, cashew nut milk, pistachio milk, and hazelnut milk had a high dispersibility improving effect when mixed with a heated liquid food or beverage.

Test Example 8. Prevention of coagulation with soy milk

Soybean milk having a specific flavor and nutrition is widely used not only as a substitute for milk but also as a material or an additive for various foods and beverages. Soybean milk with improved dispersibility can be expected to be used for new applications as well as improved quality in existing applications.

(1) Methods

To commercially available soybean milk (Manufactured by Sojasun, product name "SOJA NATURE SANS SUCRE", protein content 3.6% (w/w), raw materials: soybean, water, heat-treated), protein glutaminase "Amano" 500 (manufactured by Amano Enzyme Inc., 500 U/g) was added in an amount of 5 U or 15 U per g of soybean protein, and a reaction was caused at 50°C for 5 hours (deamidation reaction). After the enzyme reaction, the enzyme was rapidly deactivated by treatment at 90°C for 15 minutes, and cooled in flowing water, and then cooled in a refrigerator to 5°C, and thereafter, 15 mL of each was added to 150 mL of a coffee solution (pH 5.2) warmed to 90°C, and the presence or absence of coagulation was confirmed.

(2) Results

The results are shown in Table 7. As shown in Table 7, clear coagulation was observed when soy milk without enzyme treatment was added to the coffee liquid, whereas coagulation was not observed when soy milk with enzyme treatment was added to the coffee liquid. That is, the enzyme-treated soy milk has acquired the property of improving dispersibility when mixed with a high temperature liquid food or beverage.

[Table 7]

| Enzyme addition amount [U/g protein] | Presence or absence of coagulation |
| --- | --- |
| Without enzyme treatment | Coagulated |
| 5 | No coagulation |
| 15 | No coagulation |

As described above, the treatment of soy milk with the protein deamidase was also effective for preventing coagulation when soy milk is mixed with a high temperature liquid food or beverage. Therefore, as in the case of nut milk, soy milk having improved dispersibility (that is, it is difficult to coagulate) when mixed with a high temperature liquid food or beverage can be prepared by the treatment with the protein deamidase. Soybean milk having a predetermined improved dispersibility as described above is intended to be used for applications in which untreated soy milk cannot be used (or is not suitable for use) due to coagulation. Based on the above experimental results, the conditions for treating soy milk with the protein deamidase may be the same as those for nut milk.

Test Example 9. Prevention of coagulation with other plant milk

Various plant milks having a unique flavor and nutrition other than soy milk are widely used not only as a substitute for milk but also as materials and additives for various foods and beverages. The plant milk having improved dispersibility can be expected to be used for new applications as well as improved quality in existing applications.

(1) Methods

To each of commercially available pea milk (manufactured by Mighty Society, protein content: 3.2% (w/w), heat-treated), oat milk (manufactured by Liquats vegetals, protein content: 1.4% (w/w), heat-treated), hemp milk (manufactured by Ecomil, protein content: 1.0% (w/w), heat-treated), and buckwheat milk (manufactured by Natumi, protein content: 1.6% (w/w), heat-treated), was added protein glutaminase "Amano" 500 (manufactured by Amano Enzyme Inc., 500 U/g) in an amount of 1 U or 5 U per g of protein, and a reaction was caused at 50°C for 5 hours (deamidation reaction). After the enzyme reaction, the enzyme was rapidly deactivated by treatment at 90°C for 15 minutes, and cooled in flowing water, and then cooled in a refrigerator to 5°C, and thereafter, 15 mL of each was added to 150 mL of a coffee solution (pH 5.2) warmed to 90°C, and the presence or absence of coagulation was confirmed.

(2) Results

The results are shown in Table 8. As shown in Table 8, clear coagulation was observed when the plant milk without enzyme treatment was added to the coffee liquid, but no coagulation was observed when the plant milk with enzyme treatment was added to the coffee liquid. This result indicates that a plant milk in general can also have a property of improving dispersibility when mixed with a high temperature liquid food or beverage by enzyme treatment.

[Table 8]

| | Enzyme addition amount [U/g protein] | Without enzyme treatment | With enzyme treatment |
|---|---|---|---|
| Pea milk | 5 | Coagulated | No coagulation |
| Oat milk | 1 | Coagulated | No coagulation |
| Hemp milk | 5 | Coagulated | No coagulation |
| Buckwheat milk | 1 | Coagulated | No coagulation |

As described above, the treatment of various plant milks with the protein deamidase was also effective for preventing coagulation when various plant milks are mixed with a high temperature liquid food or beverage. Therefore, as in the case of nut milk, various kinds of plant milk having improved dispersibility (that is, it is difficult to coagulate) when mixed with a high temperature liquid food or beverage can be prepared by the treatment with the protein deamidase. As described above, various kinds of plant milk having predetermined improved dispersibility are used for applications in which various kinds of untreated plant milk cannot be used due to coagulation (or are not suitable for use). In addition, based on the above experimental results, the same conditions as in the case of nut milk can be adopted as the conditions of the treatment of various plant milks with the protein deamidase.

INDUSTRIAL APPLICABILITY

The present invention provides a plant milk excellent in dispersibility when added to a high temperature liquid food or beverage (beverage or liquid food) without using an additive such as an emulsifier or the like. The high dispersibility enhances the value of the plant milk itself and the liquid food or beverage using the plant milk. In addition, it is possible to provide a new liquid food or beverage that cannot be realized conventionally.
The plant milk provided by the present invention is expected to be used or applied for various uses (in particular, it is used for mixing with a high temperature acidic beverage or an acidic liquid food). It is a great advantage of the present invention that additives such as an emulsifier can be dispensed with. In addition, even in a case where a plant milk is added to high temperature coffee or the like as a substitute for milk, a special operation for preventing coagulation is not required, and thus convenience for consumers is also improved.
The present invention is not limited to the description of the embodiments and examples of the invention. Various modifications that can be easily conceived by those skilled in the art without departing from the scope of the claims are also included in the present invention. The entire contents of the articles, published patent publications, patent publications, and the like specified in this specification are incorporated herein by reference.

**Claims**

1. A plant milk treated with a protein deamidase for mixing with a heated liquid food or beverage to prepare a plant milk-containing liquid food or beverage.

2. The plant milk according to claim 1, wherein the plant milk is selected from the group consisting of nut milk, soy milk, pea milk, oat milk, hemp milk, and buckwheat milk.

3. The plant milk according to claim 2, wherein nuts as a raw material of the nut milk are selected from the group consisting of almond, cashew nut, hazelnut, pecan nut, macadamia nut, pistachio, walnut, brazil nut, peanut, coconut, chestnut, sesame, and pine nut.

4. The plant milk according to any one of claims 1 to 3, wherein a raw material plant protein is contained at a concentration of 0.2% (w/v) to 10.0% (w/v).

5. The plant milk according to any one of claims 1 to 4, wherein dispersibility when mixed with a heated liquid food or beverage is improved by the treatment.

6. The plant milk according to claim 5, wherein pH of the liquid food or beverage is 3 or more and less than 11.

7. The plant milk according to claim 6, wherein the pH of the liquid food or beverage is 5 to 7.

8. The plant milk according to claim 6, wherein the liquid food or beverage is selected from the group consisting of coffee, coffee beverages, tea, tea beverages, fruit juice, fruit juice beverages, sports beverages, nutritional beverages, soup, curry, cocoa, and chocolate beverages.

9. The plant milk according to any one of claims 1 to 8, which does not contain an emulsifier and a thickening polysaccharide for preventing coagulation.

10. The plant milk according to any one of claims 1 to 9, wherein the protein deamidase is an enzyme derived from a Chryseobacterium microorganism.

11. The plant milk of claim 10, wherein the Chryseobacterium microorganism is Chryseobacterium proteolyticum.

12. A method for producing a plant milk having improved dispersibility when mixed with a heated liquid food or beverage, the method comprising a step of treating a plant milk with a protein deamidase.

13. The production method according to claim 12, comprising the following steps (1) and (2):

(1) a step of preparing a plant milk, and
(2) a step of treating the plant milk prepared in (1) with a protein deamidase.

14. The production method according to claim 13, comprising (3) a step of performing a heating treatment after step (2).

15. A liquid food or beverage comprising the plant milk according to any one of claims 1 to 11.

16. The liquid food or beverage according to claim 15, wherein the pH is 5 or more.

17. The liquid food or beverage according to claim 15 or 16, which is selected from the group consisting of coffee beverages, coffee whiteners, tea beverages, fruit juice beverages, sports beverages, nutritional beverages, soup, curry, cocoa beverages, and chocolate beverages.

18. A method for producing a plant milk-containing liquid food or beverage, comprising a step of mixing a plant milk treated with a protein deamidase with a heated liquid food or beverage.

19. The production method according to claim 18, comprising the following steps (1) and (2):

(1) a step of preparing a plant milk treated with a protein deamidase, and
(2) a step of mixing the plant milk prepared in (1) with a heated liquid food or beverage.

## INTERNATIONAL SEARCH REPORT

| International application No. |
|---|
| **PCT/JP2021/031040** |

**A. CLASSIFICATION OF SUBJECT MATTER**

*A23C 11/10*(2021.01)i; *A23L 2/38*(2021.01)i; *A23L 7/10*(2016.01)i; *A23L 11/60*(2021.01)i; *A23L 25/00*(2016.01)i; *C12N 9/10*(2006.01)i

FI:     A23C11/10; A23L2/38 D; A23L11/60; A23L25/00; A23L7/10 Z; C12N9/10

According to International Patent Classification (IPC) or to both national classification and IPC

**B. FIELDS SEARCHED**

Minimum documentation searched (classification system followed by classification symbols)

A23C11/10; A23L2/38; A23L7/10; A23L11/60; A23L25/00; C12N9/10

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/MEDLINE/EMBASE/BIOSIS/CABA/FSTA (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2016-506732 A (OATLY AB) 07 March 2016 (2016-03-07) claims 1-22, paragraphs [0001]-[0003], [0011]-[0013], examples, table 1 | 1-19 |
| Y | claims 1-22, paragraphs [0001]-[0003], [0011]-[0013], examples, table 1 | 1-19 |
| X | WO 2017/009100 A1 (DSM IP ASSETS B. V.) 19 January 2017 (2017-01-19) claims 1-12, page 5, line 8 to page 6, example 3 | 1-19 |
| Y | claims 1-12, page 5, line 8 to page 6, example 3 | 1-19 |
| X | SUPPAVORASATIT, Inthawoot et al. Effect of Enzymatic Protein Deamidation on Protein Solubility and Flavor Binding Properties of Soymilk. Journal of Food Science, 2013, 78(1), C1-C7 abstract, introduction, fig. 1 | 1-19 |
| Y | abstract, introduction, fig. 1 | 1-19 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **30 September 2021** | **12 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
|---|
| **PCT/JP2021/031040** |

**C.    DOCUMENTS CONSIDERED TO BE RELEVANT**

| Category* | Citation of document, with indication, where appropriate, of the relevant passages | Relevant to claim No. |
|---|---|---|
| X | WU, Liang et al. Effects of Soymilk Protein Deamidation on the Properties of Soymilk. Soybean Science, 2011, 30(6), 1005-1010<br>abstract, page 1005, left column, last paragraph, 2.1 | 1-19 |
| Y | abstract, page 1005, left column, last paragraph, 2.1 | 1-19 |
| Y | WO 2009/113628 A1 (AJINOMOTO KK) 17 September 2009 (2009-09-17)<br>paragraph [0003] | 1-19 |
| Y | JP 2009-178117 A (HATTA, Hajime) 13 August 2009 (2009-08-13)<br>paragraphs [0002]-[0004] | 1-19 |
| Y | WO 2016/167269 A1 (ASAHI KASEI CORP.) 20 October 2016 (2016-10-20)<br>paragraphs [0066], [0146]-[0152] | 1-19 |
| Y | JP 58-183060 A (YUKIJIRUSHI SHOKUHIN KK) 26 October 1983 (1983-10-26)<br>page 2, upper left column | 1-19 |
| P, X | WO 2020/171106 A1 (AMANO ENZYME INC.) 27 August 2020 (2020-08-27)<br>claims 1-20, examples | 1-19 |
| P, X | WO 2020/171105 A1 (AMANO ENZYME INC.) 27 August 2020 (2020-08-27)<br>claims 1-17, examples | 1-19 |
| P, X | WO 2020/176469 A1 (AMANO ENZYME USA CO., LTD.) 03 September 2020 (2020-09-03)<br>claims 1-39, examples | 1-19 |
| P, X | WO 2021/049591 A1 (AMANO ENZYME INC.) 18 March 2021 (2021-03-18)<br>claims 1-15, examples | 1-19 |

Form PCT/ISA/210 (second sheet) (January 2015)

**INTERNATIONAL SEARCH REPORT**
Information on patent family members

International application No.

**PCT/JP2021/031040**

| Patent document cited in search report | | | Publication date (day/month/year) | Patent family member(s) | | | Publication date (day/month/year) |
|---|---|---|---|---|---|---|---|
| JP | 2016-506732 | A | 07 March 2016 | WO | 2014/123466 | A1 | |
| | | | | claims 1-23, Background of the Invention, Objects of the Invention, examples | | | |
| | | | | US | 2015/0351432 | A1 | |
| | | | | EP | 2953482 | A1 | |
| | | | | KR | 10-2015-0113200 | A | |
| | | | | CN | 105007757 | A | |
| | | | | AU | 2014215729 | A | |
| | | | | CA | 2899717 | A | |
| | | | | HK | 1212562 | A | |
| | | | | RU | 2015125371 | A | |
| | | | | SG | 11201504862Q | A | |
| WO | 2017/009100 | A1 | 19 January 2017 | (Family: none) | | | |
| WO | 2009/113628 | A1 | 17 September 2009 | US | 2011/0064847 | A1 | |
| | | | | paragraph [0006] | | | |
| | | | | EP | 2267146 | A1 | |
| | | | | CN | 102016057 | A | |
| | | | | KR | 10-2010-0127824 | A | |
| | | | | CA | 2717340 | A | |
| | | | | MX | 2010010076 | A | |
| | | | | TW | 200942616 | A | |
| JP | 2009-178117 | A | 13 August 2009 | (Family: none) | | | |
| WO | 2016/167269 | A1 | 20 October 2016 | US | 2018/0110235 | A1 | |
| | | | | paragraphs [0110], [0216]-[0228] | | | |
| | | | | EP | 3284780 | A1 | |
| | | | | CN | 107531940 | A | |
| | | | | TW | 201702303 | A | |
| JP | 58-183060 | A | 26 October 1983 | (Family: none) | | | |
| WO | 2020/171106 | A1 | 27 August 2020 | (Family: none) | | | |
| WO | 2020/171105 | A1 | 27 August 2020 | (Family: none) | | | |
| WO | 2020/176469 | A1 | 03 September 2020 | (Family: none) | | | |
| WO | 2021/049591 | A1 | 18 March 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000050887 A
- JP 2001218590 A

- WO 2006075772 A

**Non-patent literature cited in the description**

- Rich almond milk (for commercial use)'', [online], 2015. Tsukuba Dairy Products Co., Ltd, 17 August 2021